# EUROPEAN PATENT APPLICATION

(11) **EP 2 578 155 A1**
(43) Date of publication of application: **10.04.2013**
(21) Application number: 12186419.3
(22) Date of filing: 27.09.2012
(51) Int. Cl.: A61B 6/00, G09B 23/28

(54) **X-ray calibration device**

(30) Priority: 05.10.2011 US 201113253379
(71) Applicant: General Electric Company, Schenectady, NY 12345 (US)
(72) Inventor: Payne, Randall K., Madison, WI Wisconsin 53718 (US); Ergun, David L., Madison, WI Wisconsin 53718 (US); Wacker, Wynn K., Madison, WI Wisconsin 53718 (US)
(74) Representative: Bedford, Grant Richard

(57) **Abstract**

An x-ray calibration device (300) includes a core (302) including a first material with a first x-ray attenuation coefficient. The core (302) defines a cavity (306) that is configured to receive a plug of a different x-ray attenuation coefficient in order to alter the x-ray attenuation coefficient of the x-ray calibration device (300). The x-ray calibration device (300) also includes an outer layer (304) at least partially surrounding the core (302). The outer layer (304) includes a second material with a second x-ray attenuation coefficient, where the second x-ray attenuation coefficient is lower than the first x-ray attenuation coefficient.

## Description

This disclosure relates generally to an x-ray calibration device that may be used for validating visceral adipose tissue (VAT) measurements.

Characteristics of an individual, such as body weight, fat mass, height, girth, gender, age, etc. are clinical descriptors useful by physicians to predict certain health risks that may increase or decrease mortality and morbidity risk. For example, the amount or type of abdominal fat, such as subcutaneous adipose tissue (SAT) or visceral adipose tissue (VAT), are associated with, and useful predictors of, an adverse metabolic risk profile and certain diseases, such as coronary heart disease and diabetes. In addition, measuring visceral adipose tissue, for example, can relate to metabolic syndrome (i.e., a combination of medical problems that can increase the risk of heart disease and/or diabetes). People suffering from metabolic syndrome can have some or all of the following: high blood glucose, high blood pressure, abdominal obesity, low high-density lipoprotein (HDL) cholesterol, high low-density lipoprotein (LDL) cholesterol, high total cholesterol and/or high triglycerides.

Conventional methods and systems for measuring VAT are mostly performed using anthropomorphic gauges, bioimpedance gauges, weight scales, etc. These devices often are not capable of providing accurate measurements of VAT because the actual fat content is not being measured, certain assumptions/estimates are made during the calculation process, and/or the devices are not exactly calibrated. Also, reproducibility may be difficult, leading to inaccurate comparisons between examinations.

Medical diagnostic imaging systems have also been used to measure VAT content. However, the use of these systems are often costly and can expose a patient to high levels of ionizing radiation, for example, when using a computed tomography (CT) imaging system. Additionally, these imaging systems are not always available for clinical use and may have long scan times. Moreover, certain measurements are inaccurate in larger individuals.

Conventional methods and systems for determining VAT often also use simple models to approximate the abdominal cavity from an estimate of subcutaneous fat thickness measurements. However, these methods and systems often fail to accurately estimate SAT, thereby resulting in an inaccurate estimate of VAT. For example, a normal dual-energy X-ray absorptiometry (DXA) image of the abdomen is a planar two-dimensional (2D) image that cannot explicitly measure VAT because it cannot measure the thickness of SAT in the vertical plane. It has been very difficult to determine the thickness of the subcutaneous fat layer around the abdomen, especially near the buttocks, since the models used in the past do not take into account differences in the thickness of the subcutaneous fat layer around the abdomen near the buttocks.

It has been determined that VAT may be estimated based on a model used in combination with data acquired with a DXA system. However, it is still desirable to have an x-ray calibration device for validating the VAT measurements obtained with specific DXA systems. Additionally, it is desirable to have an x-ray calibration device that may be used for cross-calibration studies correlating VAT measurements obtained with a CT system and VAT estimates obtained with a DXA system. Data from cross-calibration studies between DXA and CT systems may, for instance, be used to modify the model or algorithm used for estimating VAT with a DXA system or to verify the performance of new models and/or algorithms. Since performing scans on live patients would expose the patients to unnecessary x-ray dose, particularly with respect to cross-calibration studies using CT data, there is a need for an x-ray calibration device that models the abdomen of a patient for the purpose of determining VAT measurements.

In accordance with an aspect of the disclosure, an x-ray calibration device includes a core including a first material with a first x-ray attenuation coefficient, the core defining a cavity that is configured to receive a plug of a different x-ray attenuation coefficient in order to alter the x-ray attenuation coefficient of the x-ray calibration device. The x-ray calibration device also includes an outer layer at least partially surrounding the core, the outer later comprising a second material with a second x-ray attenuation coefficient, where the second x-ray attenuation coefficient is lower than the first x-ray attenuation coefficient.

In accordance with an aspect of the disclosure, an x-ray calibration device includes a core assembly including an adjustable material that may be adjusted to provide multiple x-ray attenuation coefficients. The x-ray calibration device also includes an outer layer circumscribing the core assembly, the outer layer having an x-ray attenuation coefficient that is lower than the core assembly.

In accordance with an aspect of the disclosure, an x-ray calibration device includes a core with a generally cylindrical shape, the core including a first material with an x-ray attenuation coefficient generally equivalent to lean body tissue. The core defines two cavities. The x-ray calibration device includes an outer layer circumscribing the core, the outer layer including a second material with an x-ray attenuation coefficient generally equivalent to adipose. The x-ray calibration device includes a first plurality of plugs shaped to fit in the two cavities, wherein the first plurality of plugs include a first material with a first x-ray attenuation coefficient. The x-ray calibration device includes a second plurality of plugs shaped to fit in the two cavities, wherein the second plurality of plugs include a second material with a second x-ray attenuation coefficient. The x-ray attenuation coefficient of the x-ray calibration device may be adjusted based on the combination of the first plurality of plugs and the second plurality of plugs that are inserted into the two cavities.

Various features, aspects, and advantages will be made apparent to those skilled in the art from the accompanying drawings and detailed description thereof. In the drawings:
FIG. 1 is a diagram of an exemplary embodiment of a dual-energy X-ray image identifying an abdominal region of interest to determine an abdominal visceral adipose tissue mass of a subject;
FIG. 2 is a schematic diagram of an exemplary embodiment of a dual-energy X-ray imaging system that may be used to acquire the dual-energy X-ray image of FIG. 1;
FIG. 3 is a schematic perspective view of an x-ray calibration device according to an exemplary embodiment;
FIG. 4 is a schematic representation of a sectional view of the x-ray calibration device in accordance with an embodiment;
FIG. 5 is a schematic representation of a plurality of plugs in accordance with an embodiment; and
FIG. 6 is a schematic representation of perspective view of an x-ray calibration device in accordance with an embodiment.

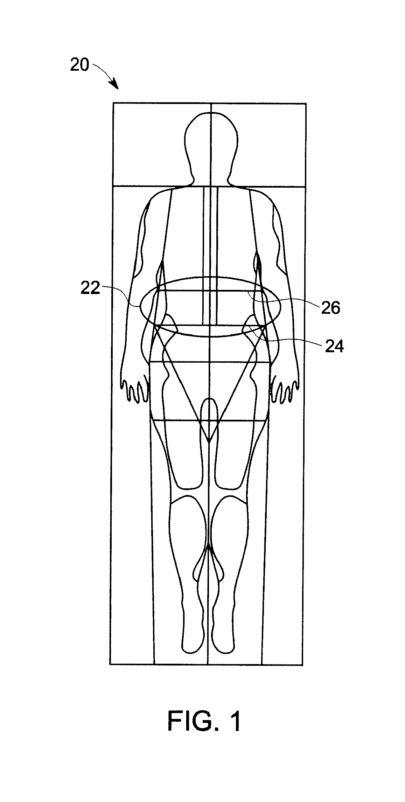

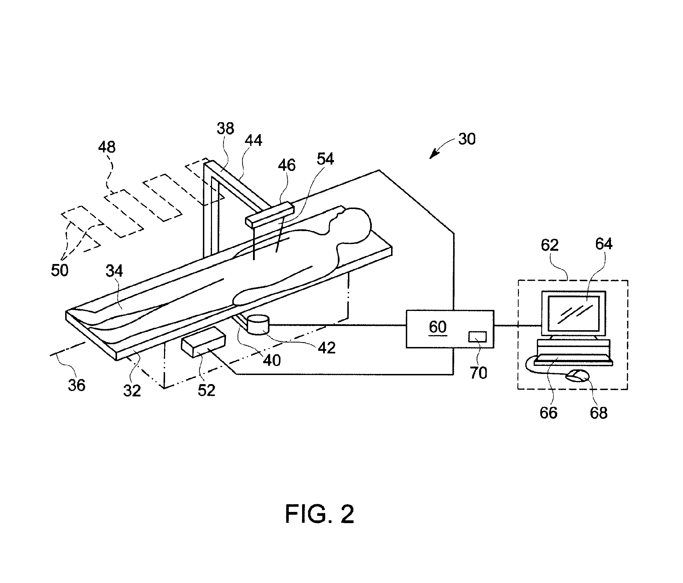

Referring now to the drawings, FIG. 1 illustrates a diagram of an exemplary embodiment of a dual-energy X-ray image 20 of a body (e.g., a subject's body), and more particularly, a full body dual-energy X-ray image 20 that may be generated from a scan of the entire body of a subject using a dual-energy X-ray imaging system. The illustrated dual-energy X-ray image 20 is a dual-energy tissue image. The image 20 is generated from a full body scan, which in some embodiments includes acquiring all bone and tissue information during a single scan, for example, a single imaging pass or operation. The total body scan may be acquired using different dual-energy X-ray imaging systems, for example, the Lunar iDXA imaging system available from GE Healthcare or other bone densitometry systems. The Lunar iDXA imaging system generally has no parallax in the transverse scanning direction. An embodiment of a dual-energy X-ray imaging system is described in more detail below in connection with FIG. 2.

The image 20 allows for the identification of different portions or regions of interest in the imaged body. For example, an android region 22 of the imaged body can be determined using the image 20. For example, based on tissue information of the imaged body, the android region 22 can be identified and the visceral adipose tissue mass thereof estimated or calculated using the acquired tissue information. A lower boundary 24 and an upper boundary 26 of the android region 22 may be determined and identified using information acquired during the dual-energy X-ray scan, including the image 20 and tissue information, including fat and lean tissue information. The android region 22 generally corresponds to the abdominal region or abdomen of a subject.

In general, the image 20 formed from the dual-energy X-ray imaging system is a two-dimensional (2D) image of a three-dimensional (3D) body. In particular, the image 20 is an anterior-posterior (AP) image of a body acquired using a dual-energy X-ray imaging system. The image 20 may also be referred to as a posterior-anterior (PA) image of the body. The dual-energy X-ray imaging system may be used to acquire both bone and tissue information (particularly soft tissue information) from one or more projection measurements of X-ray attenuation at two different X-ray energy ranges. For example, when a subject is lying down on a table between an X-ray source below the subject and an X-ray detector above the subject (or vice versa), the X-ray detector can obtain information for a dual-energy X-ray absorptiometry (DXA) AP view of the composition of the body, including tissue, as well as the spine, bones or portions thereof as a result of the passage of X-rays at two different energy levels through the subject. In an exemplary embodiment, the subject may be a human patient (i.e., a patient), an animal or an object.

FIG. 2 illustrates a schematic diagram of an exemplary embodiment of a dual-energy X-ray imaging system, illustrated as a dual X-ray absorptiometry (DXA) system 30, which may also be referred to as dual-energy bone densitometry system capable of performing bone densitometry. The system 30 is constructed in accordance with various embodiments and is configured to provide for measurement of at least soft tissue composition (body composition), including tissue thickness, an area of a bone, a length of a bone, a bone mineral content (BMC), and a bone mineral density (BMD). The BMD is calculated by dividing the BMC by the area of the bone. During operation, two X-ray beams having different energy levels are utilized to scan a subject, for example, to scan a body of a human patient (e.g., a patient) to image the body of the subject. The acquired image(s), including tissue and bone information from the imaged body, particularly determined tissue composition (body composition) and tissue thickness information, is used to calculate a visceral adipose tissue mass of the abdominal region of the subject.

The image(s) may be generated in part from determined tissue information and bone density information acquired during a dual-energy X-ray scan.

The dual-energy X-ray imaging system 30 includes a table 32 providing a horizontal surface for supporting a subject 34, for example a patient, in a supine or lateral position along a longitudinal axis 36. The dual-energy X-ray imaging system 30 also includes an imaging assembly 38, for example, such as a C-arm. The imaging assembly 38 includes a lower arm 40 that is positioned beneath the table 32 and supports an X-ray source 42 positioned at one end thereof. The imaging assembly 38 also includes an upper arm 44 that is positioned above the table 32 and supports an X-ray detector 46 at one end thereof. The imaging assembly 38 is configured such that the lower arm 40 and the upper arm 44 are coupled together so that the X-ray source 42 and the X-ray detector 46 move in unison with each other. However, it should be noted that the position of the X-ray source 42 and the X-ray detector 46 may be reversed. The X-ray detector 46 may be fabricated, for example, as a multi-element cadmium-zinc-telluride (CZT) detector providing for energy discrimination. The X-ray source 42 and the X-ray detector 46 may be moved in a transverse raster pattern 48 so as to trace a series of transverse scans 50 of the subject 34, where the X-ray source 42 and the X-ray detector 46 move from side-to-side, perpendicular to longitudinal axis 36, along the entire length of the subject 34, during which dual-energy X-ray imaging data is collected by the X-ray detector 46. Dual-energy X-ray imaging data is collected only during transverse (side-to-side) movement of the X-ray source 42 and the X-ray detector 46. The transverse raster pattern is produced by actuators (not shown) under control of a translation controller 52. During operation, the X-ray source 42 produces an X-ray fan beam 54 in a plane that is parallel to the longitudinal axis 36. However, in some embodiments, the X-ray fan beam 54 may be provided in a plane that is perpendicular to the longitudinal axis 36. The transverse raster pattern 48 is adjusted in some embodiments such that there is some overlap (e.g., slight overlap of approximately 10 percent) between successive transverse scans 50 of the X-ray fan beam 54.

In other dual-energy X-ray imaging systems, the X-ray source and the X-ray detector may be moved in a longitudinal raster pattern so as to trace a series of longitudinal scans of the subject, where the X-ray source and the X-ray detector move from head to feet and vice versa, parallel to the longitudinal axis along the entire length of the subject.

The X-ray source 42, the X-ray detector 46, and the translation controller 52 communicate with and are under the control of a computer 60 which may include both dedicated circuitry and one or more processors having the ability to execute a stored program, for example, instructions for computer 60 that are stored in memory or in software. In an exemplary embodiment, the computer 60 also includes a body fat measurement module 70. The body fat measurement module 70 utilizes the dual-energy X-ray image data, and in particular, the acquired tissue and bone information to determine an amount of visceral adipose tissue, and more particularly, abdominal visceral adipose tissue of a scanned body of the subject 34. During operation, the body fat measurement module 70 directs the dual-energy X-ray imaging system 30 to acquire a scan of a portion of the body or a full body (or total body scan), from which tissue information as well as bone information may be identified. The locations of bone landmarks may be determined automatically, manually or semi-automatically, for example, with an operator adjusting automatically generated landmarks and used to identify regions of interest of the imaged body.

The body fat measurement module 70 may then utilize the dual-energy X-ray image data, including the acquired tissue information, particularly, soft tissue information, and bone information to determine the visceral adipose tissue mass in one or more regions of interest of the subject. In various embodiments, using tissue and bone information or measurements in combination with different methods or models, abdominal visceral adipose tissue may be determined. It should be noted that different landmarks may be used to identify one or more regions of interest for which the visceral adipose tissue mass is to be determined. It also should be noted that different methods or models may be used to determine the visceral adipose tissue mass in different sections of the 2D planar image(s) from the dual-energy X-ray imaging system 30. It further should be noted that although the various embodiments are described in connection with a dual-energy X-ray imaging system, the various embodiments are not limited to a dual-energy X-ray imaging system or a particular configuration thereof.

Referring again to FIG. 2, the computer 60 communicates with a terminal, such as a workstation 62 including a display 64, a keyboard 66, and a cursor control device such as a mouse 68 allowing for operator input and the output of text and images to the operator. In some embodiments, the computer 60 is located remotely from the workstation 62. Optionally, the computer 60 may form a portion of the workstation 62. The computer 60 is adapted to perform one or more processing operations. The acquired tissue and bone information, for example, image, density and thickness information may be processed and displayed in real-time during a scanning session as the data is received. Additionally or alternatively, the data may be stored temporarily in a memory device on the computer 60 during a scanning session and then processed and displayed in an off-line operation. The information may also be stored in a long-term storage device (e.g., hard drive or server) for later access, such as during a follow-up scan of the same subject, allowing monitoring of changes in the visceral adipose tissue mass of the subject. The display 64 may include one or more monitors that present subject information, including the scanned image, which may include presenting tissue and bone information to the operator for review, diagnosis and/or analysis. The displayed images may be modified and the display settings of the display 64 also may be manually adjusted using the keyboard 66, the mouse 68, or a touch screen icon on the display 64 itself.

During operation, the dual-energy X-ray imaging system 30 may be configured to operate in either a dual-energy X-ray mode or a single energy X-ray mode. In the single energy X-ray mode, the X-ray source 42 emits X-rays in a narrow band of energies of a few keV and in the diagnostic imaging range of approximately 20-150 keV. In the dual-energy X-ray mode, the X-ray source 42 emits radiation in two or more bands of energy emitted simultaneously or in rapid succession. The X-ray source 42 may also be configured to emit a single broadband of energy of more than a few keV over the diagnostic imaging range. The dual-energy X-ray imaging system 30 may be switched between the dual-energy X-ray mode and the single energy X-ray mode by increasing or decreasing the X-ray source 42 voltage and/or current. The dual-energy X-ray imaging system 30 may also be switched between the dual-energy X-ray mode and the single energy X-ray mode by removing or adding a K-edge filter. It should be noted that the X-ray source 42 may emit X-rays at different energies or ranges of energies.

The X-ray source 42 may be configured to output a fan beam of X-rays 54 as shown in FIG. 2. The X-ray source 42 may also be configured to output a thin beam of X-rays (not shown), a cone beam of X-rays, or other configurations. In some embodiments, the body fat measurement module 70 controls the dual-energy X-ray imaging system 30 to operate in the single energy X-ray mode or dual-energy X-ray mode to acquire tissue and bone information to determine the visceral adipose tissue mass of one or more portions or regions of a subject. The dual-energy X-ray mode allows the acquisition of both tissue information and skeletal bone information, for example, soft tissue information, such as fat density or fat thickness information. Accordingly, the dual-energy X-ray mode allows for both soft tissue and skeletal imaging of the subject 34 using attenuation information from different energy levels. It should be noted that in the single energy X-ray mode, higher resolution images also may be generated.

Various embodiments provide for calculating or estimating the visceral adipose tissue mass of one or more different portions or regions of a subject. The tissue and bone information used for calculating the visceral adipose tissue mass is acquired in the various embodiments using a one or more dual-energy X-ray scans. For example, region specific X-ray scans may be performed where only a potion or region of the subject 34 (e.g., the abdominal region) is scanned for use in calculating the visceral adipose tissue mass. In other embodiments, a full body or total body X-ray scan is performed.

Visceral adipose tissue (VAT) measurements, such as those performed with either a CT system or a DXA system are attempting to determine the level of visceral adipose of a patient. The subcutaneous layer of fat is typically not included in a VAT measurement. As previously discussed, the cost and dose associated with each CT scan do not make CT a good option for VAT measurements. However, the cost and dose of DXA scans are lower than the cost and dose for CT scans.

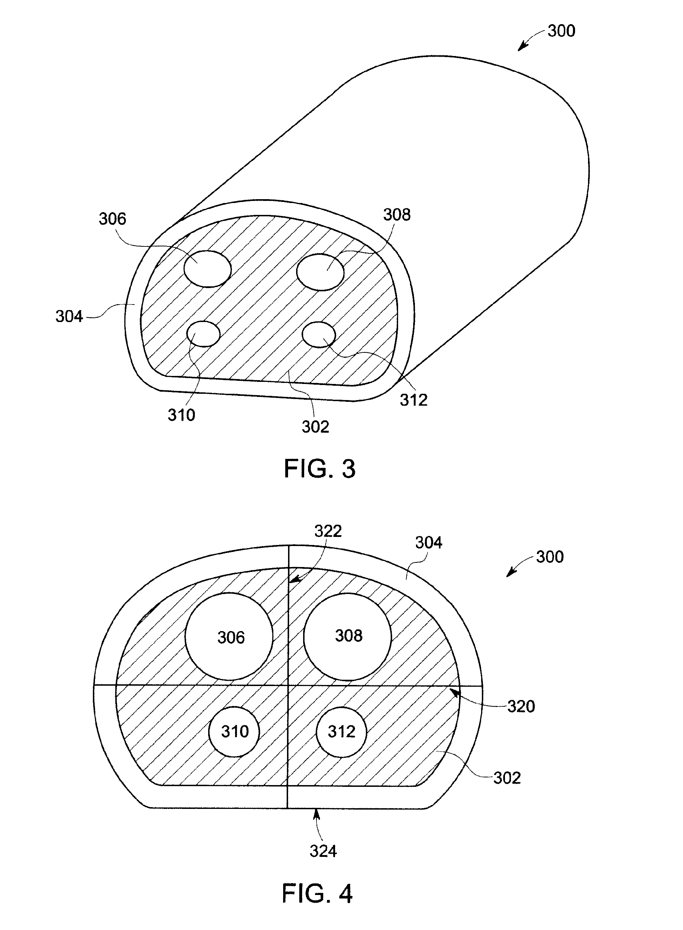

Figure 3 illustrates a schematic perspective view of an x-ray calibration device 300 according to an exemplary embodiment. The X-ray calibration device 300 may be used with a DXA system such as the DXA system 30 shown in Figure 2. The x-ray calibration device 300 includes a core 302 and an outer layer 304. The core 302 is shaped to define four cavities: a first cavity 306, a second cavity 308, a third cavity 310, and a fourth cavity 312. The core 302 is made of a material that simulates the x-ray attenuation of lean body material. In other words, the material used for the core 302 has an x-ray attenuation coefficient that is generally equivalent to that found in the lean body material of a patient. The core 302 may be generally shaped as a cylinder or as an elliptic cylinder according to some embodiments. Other embodiments may include a core with a shape that more closely matches the abdomen of a typical patient. For example, the core may have a flat side corresponding to the posterior of a patient's abdomen and the core may also have a more complicated cross section on the side corresponding to the anterior side. According to an embodiment, the lean body material used for the core 302 may have an x-ray attenuation coefficient in the range of 0% fat to 20% fat as measured by a DXA system. According to an exemplary embodiment, the core 302 may include a base material, such as polystyrene or another plastic that is modified with a higher z material, such as calcium powder, calcium phosphate, silicon, and the like, in order to obtain an x-ray attenuation coefficient in the range of 0% fat to 20% fat as measured by a DXA system. While the exemplary embodiment uses a plastic modified with a high z material, is should be appreciated by those skilled in the art that any material with the appropriate radiological properties may be used for the core 302. Using a core material with an x-ray attenuation coefficient in the range of 0-20% fat is consistent with tissue that would be considered lean when performing a DXA scan. This is due to the fact that lean body material of a patient still contains a measurable amount of fat due to cellular structures.

The x-ray calibration device 300 is shaped to model the abdomen of a typical patient. According to an exemplary embodiment, the x-ray calibration device 300 may be approximately 20 cm in length. Figure 4 is a schematic representation of a sectional view of the x-ray calibration device 300 shown in Figure 3. Common reference numbers will be used in Figures 3 and 4 to identify identical structures. According to an embodiment, the x-ray calibration device 300 may have a generally elliptical cross section, as shown in figure 4. A major axis 320 may be approximately 32 cm while a minor axis 322 may be approximately 21 cm. The x-ray calibration device 300 deviates from having a perfectly elliptical cross section in order to more closely model the anatomy of a patient's abdomen. For example, a bottom side 324 is substantially flat in a manner similar to the posterior side of the abdomen of a typical patient. Additionally having a flat bottom side 324 keeps the x-ray calibration device 300 from rocking or shifting while being scanned.

The outer layer 304 surrounds the core 302. The outer layer 304 is made of a material with an x-ray attenuation coefficient equivalent to the layer of subcutaneous fat surrounding the abdominal region of a typical patient. According to an exemplary embodiment, the outer layer 304 may comprise a material with an x-ray attenuation coefficient equivalent to a range of 70-90% fat as measure on a DXA system. The outer layer 304 circumscribes the core 302, as is shown in Figure 3 and 4.

The outer layer 304 that surrounds the core 302 has a uniform thickness according to the embodiment shown in Figures 3 and 4. However, the outer layer 304 may be of a variable thickness in order to more closely approximate a typical subcutaneous layer of fat according to other embodiments.

As previously described, the x-ray calibration device 300 defines four cavities: the first cavity 306, the second cavity 308, the third cavity 310, and the fourth cavity 312. According to an embodiment, the first cavity 306 and the second cavity 308 may both be the same shape. For example, they may both be a cylindrical shape of a first diameter. The third cavity 310 may also be the same size and shape as the fourth cavity 312. According to an embodiment, they may both be a cylinder of a second diameter that is smaller than the first diameter. All four cavities 306, 308, 310, and 312 may have a generally constant cross section for the length of the x-ray calibration device 300. Each of the cavities 306, 308, 310, and 312 may be configured to receive a plug. The cavities may be open-ended cavities in order to facilitate the easy insertion and removal of various plugs. Additional details about the cavities and the plugs will be described in detail hereinafter.

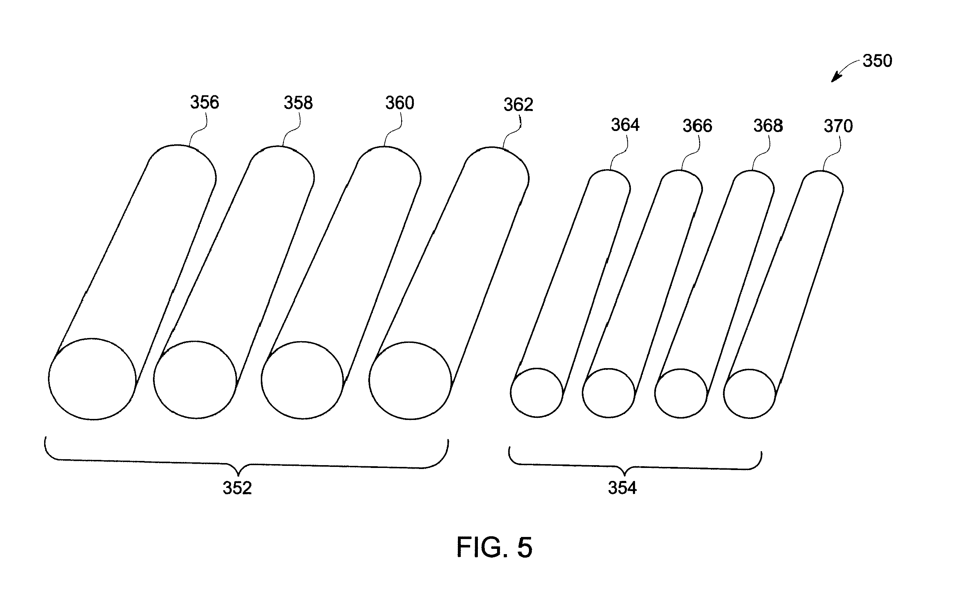

Figure 5 is a schematic representation of a plurality of plugs 350 in accordance with an embodiment. Referring to Figures 3 and 5, a first plurality of plugs 352 are sized and shaped to fit in the first cavity 306 or the second cavity 308, while the second plurality of plugs 354 are sized and shaped to fit in the third cavity 310 or the fourth cavity 312. The plugs 350 may be pressed into the cavities where there are secured via a press-fit. According to an embodiment, plugs 356 and 358 are made from a first material with a first x-ray attenuation coefficient, while plugs 360 and 362 are made from a second material with a second x-ray attenuation coefficient. Likewise, of the second plurality of plugs 354, plugs 364 and 366 are made from the first material, while plugs 368 and 370 are made from the second material. According to an embodiment, the first material may be the same material used for the core 302 and the second material may be the same material used for the outer layer 304.

The x-ray calibration device 300 may be used as a phantom for validating VAT measurements obtained at a multiple visceral adipose tissue compositions. In other words, the x-ray calibration device 300 may be scanned when performing a VAT measurement. As described with respect to Figure 5, the plugs are made of different materials, with different x-ray attenuation coefficients. The plugs may be inserted into the cavities defined by the core 302. By adjusting which of the plugs are inserted into the cavities, the overall x-ray attenuation coefficient of the x-ray calibration device 300 may be adjusted. For ease of discussion, the plugs with the same composition as the core 302 will be called "lean plugs" and the plugs with the same composition as the outer layer 304 will be called "adipose plugs." Additionally, the portion of the x-ray calibration device including the core 302 and the inserted plugs will be referred to as the "core assembly" of the x-ray calibration device 300. The core assembly is an adjustable material since it includes the core 302 and the inserted plugs. By removing one of the inserted plugs and replacing it with another plug of a different x-ray attenuation coefficient, it is easy to change the overall x-ray attenuation coefficient of the core assembly. If all four lean plugs are inserted into the cavities, then the core assembly will have an x-ray attenuation equivalent to the lean material. On the other hand, if all four adipose plugs are inserted, then the core assembly will have an x-ray attenuation coefficient equivalent to tissue with a certain percentage of adipose. The exact percentage of adipose can be determined based on geometric calculations of the volumes of the four plugs and the volume of the core 302. Additionally, any combination of adipose plugs and lean plugs may be inserted into the cavities defined by the core 302 according to an embodiment. According to other embodiments, plugs with compositions other than lean and adipose may be used. For example, plugs with compositions in between lean and adipose may be used in other embodiments. It should be appreciated that the x-ray attenuation coefficient of the core assembly may be adjusted to any one of multiple levels between the configuration where all the plugs are lean and the configuration where all the plugs are adipose. Again, since the volume and composition of each of the plugs is known, the expected results of the VAT measurements may be easily calculated and compared with the results from a CT system (not shown) or a DXA system such as the DXA system 30 (shown in Figure 2).

The x-ray calibration device 300 is well-suited for validating VAT measurements obtained with either a DXA system or a CT system for several reasons. First, the x-ray calibration device 300 models visceral tissue accurately enough to be useful as a phantom; the x-ray calibration device 300 is generally shaped like the abdomen of a patient. Second, the outer layer 304 of the x-ray calibration device 300 represents the subcutaneous layer of fat, just like what would be found on a typical patient. Therefore, the x-ray calibration device 300 may be used to confirm that a particular VAT estimation algorithm accurately accounts for subcutaneous fat. Third, since the plugs of the x-ray calibration device 300 are adapted to be easily and quickly switched out for plugs of different x-ray attenuation coefficients, the overall x-ray attenuation of the x-ray calibration device 300 may be quickly adjusted to represent a variety of known adipose levels. The user may, therefore, use the x-ray calibration device 300 to confirm CT VAT measurements and DXA VAT measurements at multiple adipose levels.

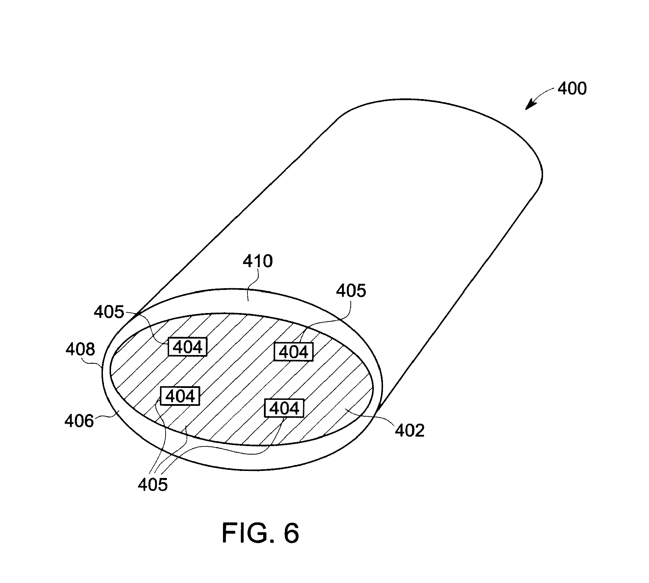

Figure 6 is a schematic representation of perspective view of an x-ray calibration device 400 according to an embodiment. The x-ray calibration device 400 includes a core 402 defining four cavities. The x-ray calibration device 400 is shown with four plugs 404 inserted into the four cavities defined by the core 402. The plugs 404 are box-shaped and they are complementary in shape to the cavities. A core assembly 405 includes both the core 402 and the plugs 404. An outer layer 406 circumscribes the core 402. The outer layer 406 of the embodiment shown in Figure 6 varies in thickness in order to more closely model the layer of subcutaneous fat on a human. For example, the outer layer 406 is thinner at point 408, corresponding to the side of a patient, than at point 410, corresponding to the anterior portion of a patient. The x-ray calibration device 400 is generally an elliptic cylinder in shape.

Other embodiments may be shaped differently than the embodiments shown in Figures 3, 4, and 6. Additionally, other embodiments may have a different number of cavities adapted to receive plugs of different x-ray attenuation levels. For example, embodiments may have a core defining as few as one cavity and embodiments may have a core defining more than four cavities. Additionally, embodiments may have plugs representing more than two x-ray attenuation coefficients. For example, by using a variety of plugs, each with a different x-ray attenuation coefficient, it is still possible to use the x-ray calibration device defining just a single cavity to model many different levels of visceral adipose.

While the disclosure has been described with reference to various embodiments, those skilled in the art will appreciate that certain substitutions, alterations and omissions may be made to the embodiments without departing from the spirit of the disclosure.

Accordingly, the foregoing description is meant to be exemplary only, and should not limit the scope of the disclosure as set forth in the following claims.

Various aspects and embodiments of the present invention are defined by the following numbered clauses:
1. An x-ray calibration device comprising:
   a core comprising a first material with a first x-ray attenuation coefficient, the core defining a cavity that is configured to receive a plug of a different x-ray attenuation coefficient in order to alter the x-ray attenuation coefficient of the x-ray calibration device; and
   an outer layer at least partially surrounding the core, the outer layer comprising a second material with a second x-ray attenuation coefficient, where the second x-ray attenuation coefficient is lower than the first x-ray attenuation coefficient.
2. The x-ray calibration device of clause 1, wherein the core is generally shaped as a cylinder.
3. The x-ray calibration device of any preceding clause, wherein the core is generally shaped as an elliptic cylinder.
4. The x-ray calibration device of any preceding clause, wherein the cavity is generally cylindrical in shape.
5. The x-ray calibration device of any preceding clause, wherein the cavity is generally box-shaped.
6. The x-ray calibration device of any preceding clause, wherein the x-ray attenuation coefficient of the core is generally equivalent to the x-ray attenuation coefficient of lean body material.
7. The x-ray calibration device of any preceding clause, wherein the second x-ray attenuation coefficient is generally equivalent to the x-ray attenuation coefficient of adipose.
8. The x-ray calibration device of any preceding clause, further comprising a plurality of plugs shaped to fit in the cavity, where each of the plurality of plugs comprises a material with a different x-ray attenuation coefficient.
9. An x-ray calibration device comprising:
   a core assembly comprising an adjustable material that may be adjusted to provide multiple x-ray attenuation coefficients; and
   an outer layer circumscribing the core assembly, the outer layer having an x-ray attenuation coefficient that is lower than the core assembly.
10. The x-ray calibration device of any preceding clause, wherein the core assembly comprises a core defining a cavity.
11. The x-ray calibration device of any preceding clause, wherein the core assembly further comprises a first plug shaped to fit within the cavity defined by the core, the first plug having an attenuation coefficient that is different than the core.
12. The x-ray calibration device of any preceding clause, wherein the core assembly further comprises a second plug shaped to fit within the cavity defined by the core, the second plug having an attenuation coefficient that is different than the first plug, wherein the x-ray attenuation coefficient of the core assembly may be adjusted based on which of the first plug or the second plug is placed in the cavity.
13. An x-ray calibration device comprising:
   a core with a generally cylindrical shape, the core comprising a first material with an x-ray attenuation coefficient generally equivalent to lean body tissue, the core defining two cavities;
   an outer layer circumscribing the core, the outer layer comprising a second material with an x-ray attenuation coefficient generally equivalent to adipose;
   a first plurality of plugs shaped to fit in the two cavities, wherein the first plurality of plugs comprise a first material with a first x-ray attenuation coefficient; and
   a second plurality of plugs shaped to fit in the two cavities, wherein the second plurality of plugs comprise a second material with a second x-ray attenuation coefficient;
   wherein the x-ray attenuation coefficient of the x-ray calibration device may be adjusted based on the combination of the first plurality of plugs and the second plurality of plugs that are inserted into the two cavities.
14. The x-ray calibration device of any preceding clause, wherein the first plurality of plugs are the same shape as the second plurality of plugs.
15. The x-ray calibration device of any preceding clause, wherein the core is generally shaped as a cylinder.
16. The x-ray calibration device of any preceding clause, wherein the core is generally shaped as an elliptic cylinder.
17. The x-ray calibration device of any preceding clause, wherein the core defines four cavities.
18. The x-ray calibration device of any preceding clause, wherein the first plurality of plugs and the second plurality of plugs are adapted to be disposed in the core with a press-fit.
19. The x-ray calibration device of any preceding clause, wherein the core defines open-ended cavities to allow for the insertion of the first plurality of plugs and the second plurality of plugs.
20. The x-ray calibration device of any preceding clause, wherein the x-ray calibration device is adapted for validating visceral adipose tissue measurements.

## Claims

1. An x-ray calibration device (300) comprising:
a core (302) comprising a first material with a first x-ray attenuation coefficient, the core (302) defining a cavity (306) that is configured to receive a plug (350) of a different x-ray attenuation coefficient in order to alter the x-ray attenuation coefficient of the x-ray calibration device (300); and
an outer layer (304) at least partially surrounding the core (302), the outer layer (304) comprising a second material with a second x-ray attenuation coefficient, where the second x-ray attenuation coefficient is lower than the first x-ray attenuation coefficient.

2. The x-ray calibration device (300) of claim 1, wherein the core (302) is generally shaped as a cylinder.

3. The x-ray calibration device (300) of any preceding claim, wherein the core (302) is generally shaped as an elliptic cylinder.

4. The x-ray calibration device (300) of any preceding claim, wherein the cavity (306) is generally cylindrical in shape.

5. The x-ray calibration device (300) of any preceding claim, wherein the cavity (306) is generally box-shaped.

6. The x-ray calibration device (300) of any preceding claim, wherein the x-ray attenuation coefficient of the core (302) is generally equivalent to the x-ray attenuation coefficient of lean body material.

7. The x-ray calibration device (300) of claim 6, wherein the second x-ray attenuation coefficient is generally equivalent to the x-ray attenuation coefficient of adipose.

8. The x-ray calibration device (300) of any preceding claim, further comprising a plurality of plugs (350) shaped to fit in the cavity (306), where each of the plurality of plugs (350) comprises a material with a different x-ray attenuation coefficient.

9. An x-ray calibration device (300) comprising:
a core assembly (405) comprising an adjustable material that may be adjusted to provide multiple x-ray attenuation coefficients; and
an outer layer (304) circumscribing the core assembly (405), the outer layer (304) having an x-ray attenuation coefficient that is lower than the core assembly (405).

10. The x-ray calibration device (300) of claim 9, wherein the core assembly (405) comprises a core (302) defining a cavity (306).

11. The x-ray calibration device (300) of claim 9 or claim 10, wherein the core assembly (405) further comprises a first plug (356) shaped to fit within the cavity (306) defined by the core (302), the first plug (356) having an attenuation coefficient that is different than the core (302).

12. The x-ray calibration device (300) of any of claims 9 to 11, wherein the core assembly (405) further comprises a second plug (360) shaped to fit within the cavity (306) defined by the core (302), the second plug (360) having an attenuation coefficient that is different than the first plug (356), wherein the x-ray attenuation coefficient of the

13. An x-ray calibration device (300) comprising:
a core (302) with a generally cylindrical shape, the core (302) comprising a first material with an x-ray attenuation coefficient generally equivalent to lean body tissue, the core (302) defining two cavities;
an outer layer (304) circumscribing the core (302), the outer layer (304) comprising a second material with an x-ray attenuation coefficient generally equivalent to adipose;
a first plurality of plugs (352) shaped to fit in the two cavities, wherein the first plurality of plugs (352) comprise a first material with a first x-ray attenuation coefficient; and
a second plurality of plugs (354) shaped to fit in the two cavities, wherein the second plurality of plugs (354) comprise a second material with a second x-ray attenuation coefficient;
wherein the x-ray attenuation coefficient of the x-ray calibration device (300) may be adjusted based on the combination of the first plurality of plugs (352) and the second plurality of plugs (354) that are inserted into the two cavities.

14. The x-ray calibration device (300) of claim 13, wherein the first plurality of plugs (352) and the second plurality of plugs (354) are adapted to be disposed in the core (302) with a press-fit.

15. The x-ray calibration device (300) of claim 13 or claim 14, wherein the core (302) defines open-ended cavities to allow for the insertion of the first plurality of plugs (352) and the second plurality of plugs (354).
